(19) **Europäisches Patentamt / European Patent Office / Office européen des brevets**

(11) **EP 2 679 165 A1**

(12) **EUROPEAN PATENT APPLICATION**
published in accordance with Art. 153(4) EPC

(43) Date of publication:
**01.01.2014 Bulletin 2014/01**

(51) Int Cl.:
*A61B 8/08* (2006.01)   *G06T 1/00* (2006.01)
*G06T 3/00* (2006.01)

(21) Application number: **12750067.6**

(22) Date of filing: **10.02.2012**

(86) International application number:
**PCT/JP2012/000920**

(87) International publication number:
**WO 2012/114670 (30.08.2012 Gazette 2012/35)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.02.2011 JP 2011036502**

(71) Applicant: **Hitachi Medical Corporation
Chiyoda-ku
Tokyo 101-0021 (JP)**

(72) Inventor: **WAKI, Koji
Tokyo 101-0021 (JP)**

(74) Representative: **Strehl Schübel-Hopf & Partner
Maximilianstrasse 54
80538 München (DE)**

(54) **ULTRASOUND DIAGNOSTIC DEVICE AND IMAGE DISPLAY METHOD**

(57)    Provided are an ultrasound diagnostic device and an image display method, with which a luminosity correction of a cross-section image is carried out in a superimposed region of the cross-section image and an elastic image. An ultrasound diagnostic device comprises: an ultrasound probe (12); a cross-section image configuration unit (20) which configures a cross-section image which is configured by a reflected echo signal which is received via the ultrasound probe (12); an elastic image configuration unit (34) which configures an elastic image on the basis of elasticity information which is computed from the reflected echo signal; and an image display unit (26) which displays a superimposed image from the cross-section image and the elastic image. The ultrasound diagnostic device further comprises: a cross-section image correction unit (40) which corrects the brightness of the cross-section image in a superimposed region of the cross-section image and the elastic image.

FIG. 1

EP 2 679 165 A1

**Description**

Technical Field

The present invention relates to an ultrasonic diagnostic device and an image display method for displaying a tomographic image concerning an image pickup target region in an object using ultrasound and displaying an elasticity image indicating hardness or softness of a biological tissue.

Background Art

An ultrasonic diagnostic device transmits ultrasound to an inside of an object using an ultrasonic probe, receives a reflected echo signal of the ultrasound corresponding to structure of a biological tissue from the inside of the object, and forms and displays a tomographic image.
In recent years, a method of converting elasticity information, i.e., hardness information into an image according to displacement calculated from a reflected echo signal of ultrasound has been developed. A tomographic image and an elasticity image are alternately formed, superimposed, and displayed (superimposing display) (Patent Literature 1).

Citation List

Patent Literature

     Patent Literature 1: JP-A-2004-141505

Summary of Invention

Technical Problem

However, the above patent literature discloses that the tomographic image and the elasticity image are superimposed to display a superimposed image but does not describe that, when the tomographic image and the elasticity image are superimposed to display the superimposed image, luminance of the tomographic image superimposed on the elasticity image is corrected.
Therefore, it is likely that textures of form information of the tomographic image and form information of the tomographic image in a superimposed region for the superimposed image of the tomographic image and the elasticity image are different and the form information of the tomographic image in the superimposed region cannot be appropriately identified.
It is an object of the present invention to improve visibility of the form information of the tomographic image in the superimposed region for the tomographic image and the elasticity image.

Solution to Problem

In order to attain the above object, the present invention provides an ultrasonic diagnostic device including: an ultrasonic probe; a tomographic image forming unit configured to form a tomographic image according to a reflected echo signal received via the ultrasonic probe; an elasticity image forming unit configured to form an elasticity image on the basis of elasticity information calculated according to the reflected echo signal; and an image displaying unit configured to display a superimposed image formed by the tomographic image and the elasticity image, the ultrasonic diagnostic device including a tomographic image correcting unit configured to correct luminance of the tomographic image in a superimposed region for the tomographic image and the elasticity image. The tomographic image correcting unit enhances the tomographic image in the superimposed region and diminishes the tomographic image in the superimposed region. The tomographic image correcting unit changes a luminance distribution of the tomographic image in the superimposed region on the basis of a correction parameter for contributing a change of the luminance distribution of the tomographic image in the superimposed region.
The ultrasonic diagnostic device further includes a superimposed region detecting unit configured to detect the superimposed region for the tomographic image formed by the tomographic image forming unit and the elasticity image formed by the elasticity image forming unit. The superimposed region detecting unit detects, from position information concerning a display region of the tomographic image and position information concerning a display region of the elasticity image, the superimposed region that is a region where the tomographic image and the elasticity image are superimposed.

By performing the luminance correction of the tomographic image in the superimposed region for the superimposed image of the tomographic image and the elasticity image, it is possible to improve visibility of form information of the tomographic image.

Advantageous Effects of Invention

According to the present invention, it is possible to improve visibility of form information of the tomographic image in the superimposed region for the tomographic image and the elasticity image.

Brief Description of Drawings

[Figure 1] Figure 1 is a diagram showing a configuration of an ultrasonic diagnostic device according to the present invention.
[Figure 2] Figure 2 is a diagram showing an image generating method of the ultrasonic diagnostic device according to the present invention.
[Figure 3] Figure 3 is a diagram showing correction of a tomographic image of a tomographic image correcting unit 40 according to the present invention.
[Figure 4] Figure 4 is a diagram showing correction of the tomographic image of the tomographic image correcting unit 40 according to the present invention.
[Figure 5] Figure 5 is a diagram showing a display form of an image display unit 26 according to the present invention.
[Figure 6] Figure 6 is a diagram showing a display form of the image display unit 26 according to the present invention.
[Figure 7] Figure 7 is a diagram showing a display form of the image display unit 26 according to the present invention.
[Figure 8] Figure 8 is a diagram for explaining second and third embodiments according to the present invention.

Description of Embodiments

An ultrasonic diagnostic device to which the present invention is applied is explained using the figures.

(First Embodiment)

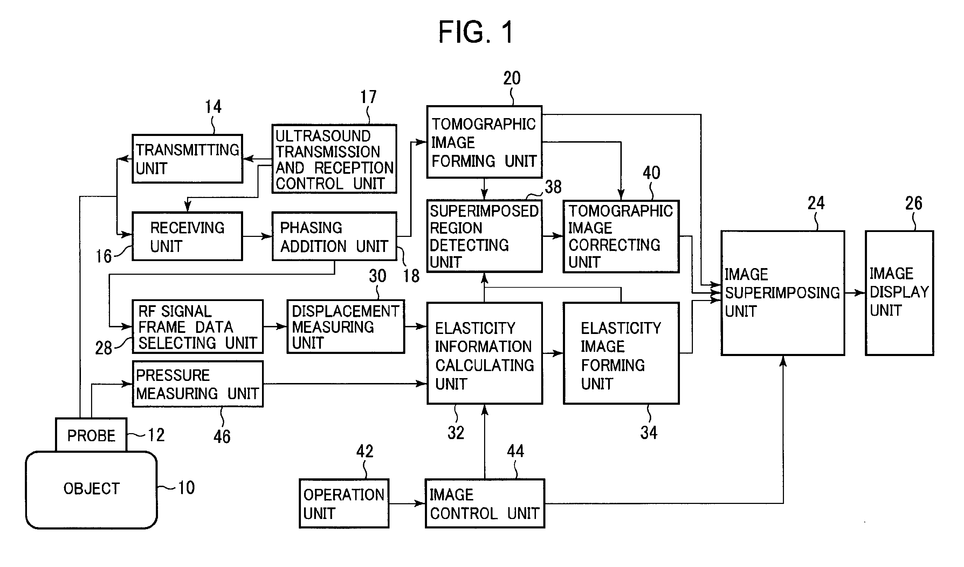

Figure 1 is a block diagram showing a configuration of an ultrasonic diagnostic device to which the present invention is applied. As shown in Figure 1, an ultrasonic diagnostic device 1 according to this embodiment includes an ultrasonic probe 12, a tomographic image forming unit 20 configured to form a tomographic image according to a reflected echo signal received via the ultrasonic probe, an elasticity image forming unit 34 configured to form an elasticity image on the basis of elasticity information calculated according to the reflected echo signal, and an image display unit 26 configured to display a superimposed image formed by the tomographic image and the elasticity image and includes a tomographic image correcting unit 40 configured to correct luminance of the tomographic image in a superimposed region for the tomographic image and the elasticity image. In an image display method according to this embodiment, a tomographic image is formed according to a reflected echo signal received via an ultrasonic probe, an elasticity image is formed on the basis of elasticity information calculated according to the reflected echo signal, a superimposed image formed by the tomographic image and the elasticity image is displayed, and luminance of the tomographic image in a superimposed region for the tomographic image and the elasticity image is corrected.
The ultrasonic diagnostic device according to this embodiment is specifically explained. As shown in Figure 1, the ultrasonic diagnostic device includes the ultrasonic probe 12 used in contact with an object 10, a transmitting unit 14 configured to repeatedly transmit ultrasound to the object 10 via the ultrasonic probe 12 at a time interval, a receiving unit 16 configured to receive, as a reflected echo signal, the ultrasound reflected from the object 10, an ultrasound transmission and reception control unit 17 configured to control the transmitting unit 14 and the receiving unit 16, and a phasing addition unit 18 configured to subject the reflected echo received by the receiving unit 16 to phasing addition.
The ultrasonic diagnostic device includes the tomographic image forming unit 20 configured to form a tomographic image (e.g., a monochrome tomographic image) of the object 10 on the basis of RF signal frame data from the phasing addition unit 18, an RF signal frame data selecting unit 28 configured to store the RF signal frame data output from the phasing addition unit 18 and select at least two RF signal frame data, a displacement measuring unit 30 configured to measure displacement of a biological tissue of the object 10 using the RF signal frame data, an elasticity information calculating unit 32 configured to calculate elasticity information from the displacement measured by the displacement measuring unit 30, and the elasticity image forming unit 34 configured to form a color elasticity image from the elasticity information calculated by the elasticity information calculating unit 32. The elasticity information is any one of distortion, a modulus of elasticity, a coefficient of viscosity, and displacement. Further, the ultrasonic diagnostic device includes an image superimposing uniting unit 24 configured to adjust the tomographic image and the elasticity image to match the display of the image display unit 26 and superimpose the tomographic image and the elasticity image or display the tomographic image and the elasticity image in parallel to superimposing unite the tomographic image and the elasticity image and the image display unit 26 configured to display an image output from the image superimposing uniting unit 24.

Further, the ultrasonic diagnostic device includes a superimposed region detecting unit 38 configured to detect a superimposed region for the tomographic image formed by the tomographic image forming unit 20 and the elasticity image formed by the elasticity image forming unit 34 and a tomographic image correcting unit 40 configured to correct the tomographic image in the superimposed region for the tomographic image and the elasticity image detected by the superimposed region detecting unit 38.

Further, the ultrasonic diagnostic device includes an operation unit 42 for giving an instruction to an image control unit 44 and the image control unit 44 configured to communicate control information based on the instruction from the operation unit 42 to the elasticity information calculating unit 32 and the image superimposing uniting unit 24.

Here, each of the components of the ultrasonic diagnostic device is explained more in detail. The ultrasonic probe 12 is formed by disposing a plurality of oscillators. The ultrasonic probe 12 has a function of transmitting and receiving ultrasound to and from the object 10 via the oscillator.

The transmitting unit 14 has a function of generating a wave transmission pulse for driving the ultrasonic probe 12 to generate ultrasound and setting a convergent point of the ultrasound to be transmitted to certain depth. The receiving unit 16 amplifies, with a predetermined gain, a reflected echo signal based on the ultrasound received by the ultrasonic probe 12 and generates an RF signal, i.e., a wave reception signal. The phasing addition unit 18 receives the RF signal amplified by the receiving unit 16, subjects the RF signal to phase control, forms an ultrasonic beam with respect to one or a plurality of convergent points, and generates RF signal frame data.

The tomographic image forming unit 20 receives the RF signal frame data from the phasing addition unit 18, performs signal processing such as gain correction, log compression, wave detection, edge enhancement, and filter processing and obtains a tomographic image.

The RF signal frame data selecting unit 28 stores a plurality of RF signal frame data from the phasing addition unit 18 and selects one set of, i.e., two RF signal frame data from the stored RF signal frame data group. For example, RF signal frame data generated in time series, i.e., on the basis of a frame rate of an image from the phasing addition unit 18 is sequentially  stored in the RF signal frame data selecting unit 28. The RF signal frame data selecting unit 28 selects stored RF signal frame data (N) as first data and, at the same time, selects one RF signal frame data (X) out of an RF signal frame data group (N-1, N-2, N-3 ... N-M) stored temporally in the past. Here, N, M, and X are index numbers affixed to the RF signal frame data and are natural numbers.

The displacement measuring unit 30 performs one-dimensional or two-dimensional correlation processing or the like from the selected one set of RF signal frame data (N) and RF signal frame data (X) and calculates a one-dimensional or two-dimensional displacement distribution concerning displacement and moving vectors, i.e., directions and magnitudes of the displacement in the biological tissue corresponding to respective points of the tomographic image. Here, a block matching method is used for detection of the moving vectors. The block matching method is a method of dividing an image into blocks formed by, for example $N \times N$ pixels and, focusing on the block in a region of interest, finding a block most approximate to the focused block from a preceding frame, and determining a sampled value through predictive coding, i.e., differential referring to the found block.

The elasticity information calculating unit 32 calculates distortions or moduli of elasticity of the biological tissue corresponding to the respective points on the tomographic image from a measurement value, for example, the displacement output from the displacement measuring unit 30 and a pressure value output from a pressure measuring unit 46. A reference plasmodium (not shown in the figure) can be set on a surface of the ultrasonic probe 12 and the pressure measuring unit 46 can measure a pressure value from a degree of deformation of the reference plasmodium deformed by pressing the object 10.

At this point, the distortion is calculated by subjecting, for example, the displacement of the biological tissue to space differential. The modulus of elasticity is calculated by dividing a change in pressure by a change in distortion. For example, when displacement measured by the displacement measuring unit 30 is represented as L(X) and pressure measured by the pressure measuring unit 46 is represented as P(X), distortion $\Delta S(X)$ can be calculated by subjecting L(X) to space differential. Therefore, the distortion $\Delta S(X)$ is calculated using an equation $\Delta S(X) = \Delta L(X)/\Delta X$. A modulus of elasticity Ym(X) is calculated by an equation $Ym = (\Delta P(X))/\Delta S(X)$. Moduli of elasticity of the biological tissue equivalent to the respective points of the  tomographic image are calculated from the modulus of elasticity Ym. Therefore, two-dimensional elasticity images can be continuously obtained. Note that the modulus of elasticity is a ratio to simple tensile stress applied to an object and distortion that occurs in parallel to stretch.

The elasticity image forming unit 34 forms a two-dimensional elasticity image on the basis of the elasticity information of the biological tissue at the respective measurement points calculated by the elasticity information calculating unit 32.

The operation unit 42 includes a keyboard including various keys and a track ball or the like. The track ball of the operation unit 42 is rotated, whereby, for example, the image control unit 44 adjusts a range of a superimposed region for a superimposed image. A decision key of the keyboard of the operation unit 42 is pressed, whereby the image control unit 44 decides the adjusted superimposed region. The image control unit 44 communicates set position information concerning the superimposed region to the elasticity information calculating unit 32 and the image superimposing uniting unit 24. The image superimposing uniting unit 24 displays a contour line based on the superimposed region to be superimposed on the tomographic image or the superimposed image.

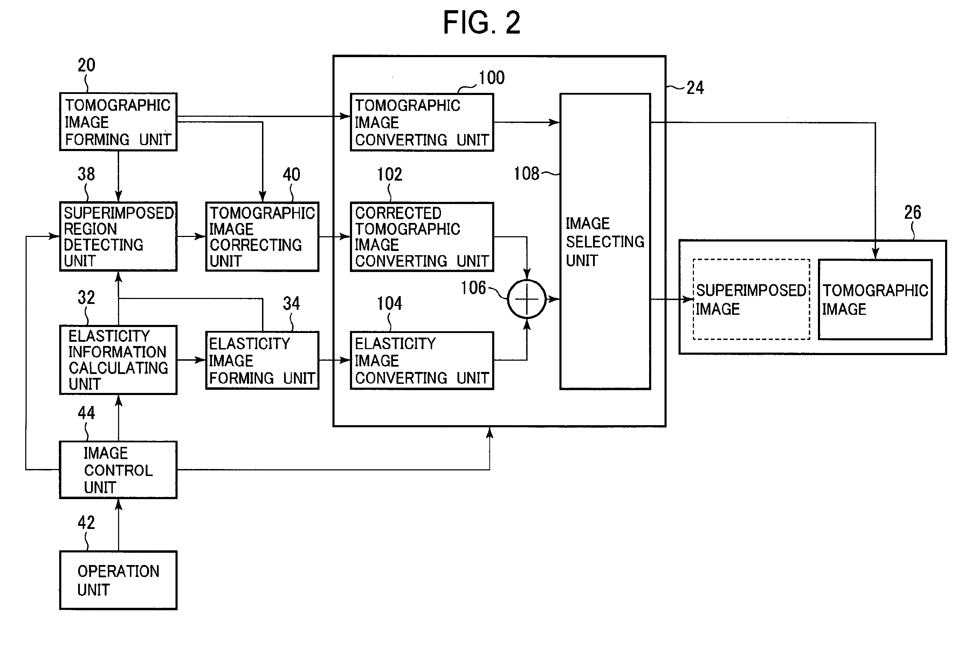

Here, explanation is made referring to Figure 2. Figure 2 is a diagram for explaining an image generating method for a tomographic image and a superimposed image of the tomographic image and an elasticity image.

The image superimposing uniting unit 24 includes a tomographic image converting unit 100 configured to convert a tomographic image output from the tomographic image forming unit 20 into monochrome information, a corrected tomographic image converting unit 102 configured to convert the corrected tomographic image output from the tomographic image correcting unit 40 into hue information, an elasticity image converting unit 104 configured to convert an elasticity image output from the elasticity image forming unit 34 into hue information, an adding unit 106 configured to add up the corrected tomographic image and the elasticity image at a predetermined superimposition ratio to form a superimposed image, and an image selecting unit 108 configured to select the superimposed image and the tomographic image.

The tomographic image converting unit 100 has a function of converting the tomographic image output from the tomographic image forming unit 20 into monochrome information. The tomographic image converting unit 100 converts the tomographic image output from the tomographic image forming unit 20 into the three primary colors of light, i.e., red (R), green (G), and blue (B) and converts the tomographic image into a monochrome tomographic image. The tomographic image converting unit 100 has a scan converter function for matching the tomographic image to display of the image display unit 26.

The superimposed region detecting unit 38 is connected to the tomographic image forming unit 20 and the elasticity information calculating unit 32, the elasticity image forming unit 34, or the image control unit 44. The superimposed region detecting unit 38 obtains position information concerning a display region of the tomographic image from the tomographic image forming unit 20. The superimposed region detecting unit 38 obtains position information concerning a display region of the elasticity image from the elasticity information calculating unit 32, the elasticity image forming unit 34, or the image control unit 44.

The superimposed region detecting unit 38 detects a superimposed region, which is a region where the tomographic image and the elasticity image are superimposed, from the position information concerning the display region of the tomographic image and the position information concerning the display region of the elasticity image. Specifically, the superimposed region detecting unit 38 detects, from a two-dimensional coordinate of the display region of the tomographic image and a two-dimensional coordinate of the display region of the elasticity image, for each of coordinates, whether a pixel of the tomographic image and a pixel of the elasticity image are superimposed. A two-dimensional coordinate of the superimposed region detected by the superimposed region detecting unit 38 is calculated. The two-dimensional coordinate of the superimposed region is communicated to the tomographic image correcting unit 40.

The tomographic image correcting unit 40 corrects the tomographic image in the superimposed region for the superimposed image detected by the superimposed region detecting unit 38. Specifically, the tomographic image correcting unit 40 enhances luminance of the tomographic image in the superimposed region and diminishes luminance of the tomographic image in the superimposed region. For example, the tomographic image correcting unit 40 changes a luminance distribution of the tomographic image in the superimposed region on the basis of a correction parameter for contributing a change of the luminance distribution of the tomographic image in the superimposed region.

Like the function of the tomographic image converting unit 100, the corrected tomographic image converting unit 102 converts the corrected tomographic image output from the tomographic image correcting unit 40 into the three primary colors of light, i.e., red (R), green (G), and blue (B). The corrected tomographic image converting unit 102 has a scan converter function for matching the corrected tomographic image to the display of the image display unit 26.

The elasticity image converting unit 104 has a function of giving hue information to the elasticity image from the elasticity image forming unit 34. The elasticity image converting unit 104 converts the elasticity image into the three primary colors of light, i.e., red (R), green (G), and blue (B). For example, the elasticity image converting unit 104 converts elasticity data with large distortion into a red code and, at the same time, converts elasticity data with small distortion into a blue code.

The adding unit 106 adds up the tomographic image and the elasticity image at a predetermined superimposition ratio to form a superimposed image. The adding unit 106 adds up the converted tomographic image and the elasticity image at a superimposition ratio $\alpha$ and superimposing unites the tomographic image and the elasticity image. Here, the superimposition ratio $\alpha$ is arbitrarily set in advance from the operation unit 42 by an operator according to characteristics and the like of the biological tissue and is a value larger than zero and smaller than one. When this superimposition ratio α is used, superimposed images to be generated are superimposing united as indicated by Math. 1.
{Math. 1}

(Superimposed image R)=(1-α)×(tomographic image

R)+α×(elasticity image R)

(Superimposed image G)=(1-α)×(tomographic image

G)+α×(elasticity image G)

(Superimposed image B)=(1-α)×(tomographic image

B)+α×(elasticity image B)

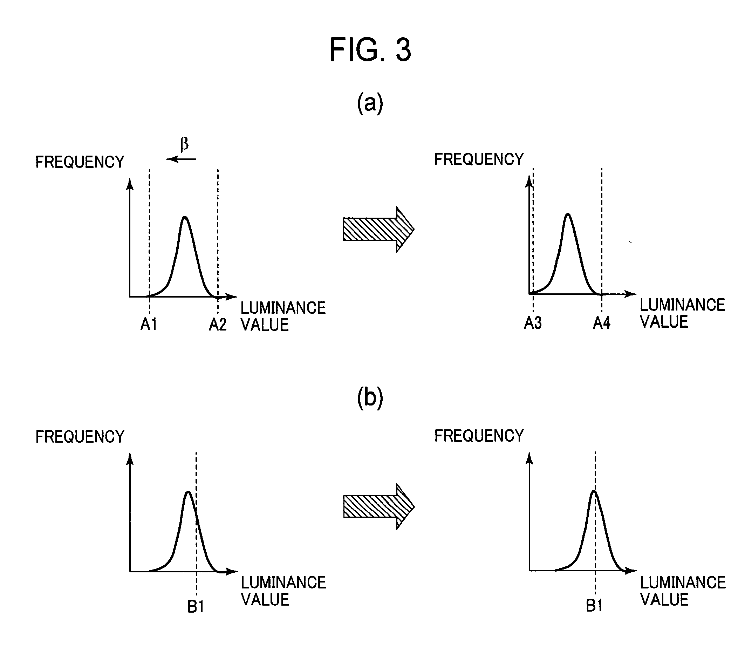

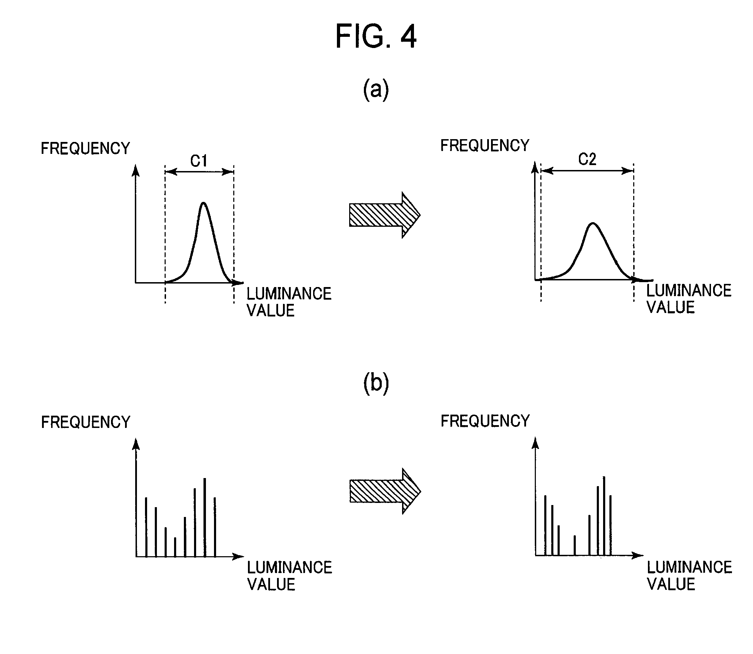

The superimposed images superimposed by the adding unit 106 are output to the image selecting unit 108.
The image selecting unit 108 selects an image to be displayed on the image display unit 26 out of the tomographic image and the superimposed image. According to the selection by the image selecting unit 108, the image display unit 26 can display the tomographic image or the superimposed image and, for example, can display the tomographic image and the superimposed image in parallel.
A specific example of the correction of the tomographic image by the tomographic image correcting unit 40 is explained using Figures 3 and 4. Left figures of Figures 3(a) and (b) and Figures 4(a) and (b) show relations between a luminance value and a frequency of an uncorrected tomographic image. Right figures of Figures 3(a) and (b) and Figures 4(a) and (b) show relations between a luminance value and a frequency of a corrected tomographic image.
Figure 3(a) is a diagram showing a method of setting a correction luminance value of a luminance distribution of the tomographic image in the superimposed region for the superimposed image of the elasticity image and the tomographic image to thereby performing correction for enhancing or diminishing the tomographic image in the superimposed region. The correction luminance value is a correction parameter for contributing a change of the luminance distribution of the tomographic image in the superimposed region. The tomographic image correcting unit 40 performs correction of the tomographic image in the superimposed region on the basis of the correction luminance value for contributing a change of the luminance distribution of the tomographic image in the superimposed region.
First, a correction luminance value β of the luminance distribution of the tomographic image in the superimposed region is set by the operation unit 42. The set correction luminance value β is communicated to the tomographic image correcting unit 40 via the image control unit 44. The tomographic image correcting unit 40 performs correction of the tomographic image in the superimposed region using Math. 2.
{Math. 2}

$$\text{Output} = \text{Input} + \beta$$

Output: Luminance value of the tomographic image after correction
Input: Luminance value of the tomographic image
β: Correction luminance value

As shown in Figure 3(a), the tomographic image correcting unit 40 performs correction for shifting the luminance distribution of the tomographic image in the superimposed region according to the correction luminance value β. Here, luminance distributions A1 to A2 in the tomographic image in the superimposed region are shifted by the tomographic image correcting unit 40 in a direction in which the luminance value is small (a left direction) by the correction luminance value β and the tomographic image is corrected to have luminance distributions A3 to A4. Luminance distribution widths of the luminance distributions A1 to A2 of the tomographic image and the luminance distributions A3 to A4 of the tomographic image are the same. The luminance distribution is shifted while the width of the luminance distribution of the tomographic image is kept.

In this way, the luminance distribution of the tomographic image in the superimposed region is the luminance distributions A3 to A4. Since the luminance distribution of the tomographic image in the superimposed region is set low according to the correction luminance value $\beta$, the luminance of the tomographic image in the superimposed region decreases.

The operator can adjust the correction luminance value $\beta$ with the operation unit 42 while observing the tomographic image in the superimposed region with the image display unit 26. The operator can set the luminance distribution of the tomographic image in the superimposed region high or can set the luminance of the tomographic image in the superimposed region high according to the correction luminance value $\beta$.

Figure 3(b) is a diagram showing a method of setting a peak reference value in the luminance distribution of the tomographic image to thereby perform correction for enhancing or diminishing the tomographic image in the superimposed image. The peak reference value is a correction parameter for contributing a change of the luminance distribution of the tomographic image in the superimposed region and is an index value for adjusting a position of a peak in the luminance distribution of the tomographic image.

First, a peak reference value B1 of the tomographic image in the superimposed region is set by the operation unit 42. The set peak reference value B1 is communicated to the tomographic image correcting unit 40 via the image control unit 44.

As shown in Figure 3(b), the tomographic image correcting unit 40 performs correction for shifting the luminance distribution of the tomographic image in the superimposed region according to the peak reference value B1. Here, the luminance distribution in the tomographic image in the superimposed region is shifted by the tomographic image correcting unit 40 in a direction in which the luminance value is large (a right direction) and the tomographic image is corrected to match a peak of the luminance distribution to the peak reference value B1. Luminance distribution widths of the luminance distribution of the tomographic image before the shift and the luminance distribution of the tomographic image after the shift are the same. The luminance distribution is shifted while the width of the luminance distribution of the tomographic image is kept. Since the peak reference value B1 in the luminance distribution of the tomographic image is set to high luminance, a luminance distribution of the tomographic image in the superimposed region can be set high. The luminance of the tomographic image in the superimposed region increases and the tomographic image is enhanced.

If the peak reference value B1 in the luminance distribution of the tomographic image is set to low luminance, the luminance of the tomographic image in the superimposed region is suppressed and the tomographic image is diminished.

The tomographic image correcting unit 40 can also perform correction for enhancing or diminishing the tomographic image in the superimposed region on the basis of the luminance information of the tomographic image. For example, the tomographic image correcting unit 40 sets a luminance reference value A set in advance, calculates an average luminance B of the tomographic image from the tomographic image forming unit 20 and calculates a ratio (A/B) of A and B. The tomographic image correcting unit 40 multiplies the tomographic image in the superimposed region with a ratio of the luminance reference value and the average luminance value. Consequently, the tomographic image in the superimposed region can be automatically adjusted.

In order to perform correction for enhancing or diminishing the tomographic image in the superimposed region on the basis of the luminance information of the tomographic image, the tomographic image correcting unit 40 can automatically correct the luminance of the tomographic image to be rather dark when the luminance of the tomographic image in the superimposed region is too high and can automatically correct the luminance of the tomographic image to be rather bright when the luminance of the tomographic image in the superimposed region is too low.

Figure 4(a) is a diagram showing a method of adjusting luminance distribution width in the luminance distribution of the tomographic image to thereby perform correction for enhancing or diminishing the tomographic image in the superimposed image.

There is luminance distribution width C1 of the tomographic image in the superimposed region. Luminance distribution width C2 of the tomographic image in the superimposed region is set by the operation unit 42. The set luminance distribution width C2 is communicated to the tomographic image correcting unit 40 via the image control unit 44. The luminance distribution width is a correction parameter for contributing a change of the luminance distribution of the tomographic image in the superimposed region.

As shown in Figure 4(a), the tomographic image correcting unit 40 performs correction for changing the luminance distribution of the tomographic image in the superimposed region according to the luminance distribution width C2. Here, the tomographic image is corrected by the tomographic image correcting unit 40 such that the luminance distribution width C1 of the tomographic image in the superimposed region changes to the luminance distribution width C2. At this point, an area of the luminance distribution at the luminance distribution width C1 of the tomographic image and an area of the luminance distribution at the luminance distribution width C2 of the tomographic image are the same.

Since the luminance distribution of the tomographic image in the superimposed region is set in a wide range, it is possible to increase gradation of the luminance distribution of the tomographic image in the superimposed region.

Therefore, it is possible to enhance the tomographic image in the superimposed region.

The tomographic image correcting unit 40 can also correct the tomographic image in the superimposed image by adjusting a correction coefficient γ according to the following equation. The correction coefficient γ is a correction parameter for contributing a change of the luminance distribution of the tomographic image in the superimposed region. When the correction coefficient γ is set large, the luminance of the tomographic image in the superimposed region is suppressed. When the correction coefficient γ is set small, the luminance of the tomographic image in the superimposed region increases.

{Math. 3}

$$\text{Output}=65536\times(\text{Input}\div65536)\gamma$$

Output: Luminance value of the tomographic image after correction
Input: Luminance value of the tomographic image
γ: Correction coefficient

If the gradation of the tomographic image in the superimposed region is adjusted in this way, it is possible to increase transparency of the tomographic image in the superimposed region and more clearly display an internal structure of form information of the tomographic image.

Figure 4(b) is a diagram showing a method of setting density in the luminance distribution of the tomographic image to thereby perform correction for enhancing or diminishing the tomographic image in the superimposed image.

The tomographic image correcting unit 40 performs correction of the tomographic image according to the luminance distribution of the tomographic image in the superimposed region. As shown in Figure 4(b), the tomographic image correcting unit 40 corrects the luminance value to increase density of the luminance value of the peak of the luminance distribution in the superimposed image and reduce density of the luminance values in places other than the peak.

The tomographic image correcting unit 40 can also correct the tomographic image to enhance a contour of the tomographic image in the superimposed region. Specifically, the tomographic image correcting unit 40 adjusts a filter coefficient for extracting a highfrequency component or a low-frequency component of the tomographic image in the superimposed region. It is possible to increase transmission in only a region of attention by enhancing streaks in the inside of the tomographic image and a contour of a tumor part in the superimposed region. A method of enhancing the contour of the tomographic image in the superimposed region is represented by methods such as a relaxation method, a zero crossing method, and a Canny method. As the method, there is a method of further enhancing a boundary portion of the tomographic image in the superimposed region using boundary information obtained by spatially differentiating the tomographic image.

Note that the image display unit 26 can also display correction parameters such as the correction luminance value and the correction coefficient. The operator can check a correction degree of the tomographic image in the superimposed region.

The tomographic image correcting unit 40 can also set a correction parameter for the tomographic image in the superimposed region on the basis of the superimposition ratio α of the tomographic image and the elasticity image in the superimposed region. For example, when the superimposition ratio α is high ($0.5<\alpha<1$), the elasticity image is enhanced. At this point, the tomographic image correcting unit 40 sets the luminance distribution of the tomographic image in the superimposed region low according to the correction luminance value β. Therefore, the luminance of the tomographic image in the superimposed region is suppressed. When the superimposition ratio α is low ($0<\alpha<0.5$), the elasticity image is diminished. At this point, the tomographic image correcting unit 40 sets the luminance distribution of the tomographic image in the superimposed region high according to the correction luminance value β. Therefore, the luminance of the tomographic image in the superimposed region is enhanced.

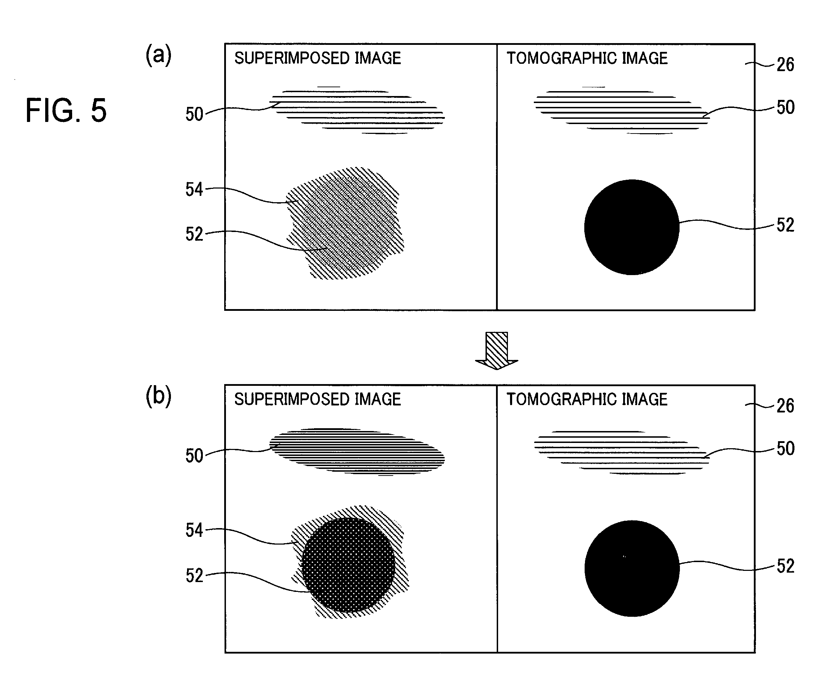

Next, a display form of the image display unit 26 is explained using Figures 5 to 7. Figure 5 shows a display form of a superimposed image in which a display region of a tomographic image and a display region of an elasticity image are the same. When the display region of the tomographic image and the display region of the elasticity image are the same, the display region of the tomographic image is a superimposed region for the superimposed image as it is. When the display region of the tomographic image and the display region of the elasticity image are the same, the superimposed region detecting unit 38 detects position information concerning the display region of the tomographic image as the superimposed region for the superimposed image. The tomographic image correcting unit 40 performs correction of the tomographic image in the superimposed region according to any one of the above methods shown in Figure 3, Figure 4, and the like.

Figure 5(a) shows a display form for displaying a superimposed image formed by an uncorrected tomographic image and an elasticity image and the tomographic image in parallel. Figure 5(b) shows a display form for displaying a superimposed image formed by a corrected tomographic image and an elasticity image and the tomographic image in parallel.

In the tomographic image shown in Figure 5(a), a normal tissue 50 and a tumor tissue 52, which are form information, are displayed. In the superimposed image shown in Figure 5(a), in addition to the normal tissue 50 and the tumor tissue 52, which are the form information, a hardened tissue 54, which is hardness information in the elasticity image, is displayed to be superimposed on the tumor tissue 52 in the tomographic image.

In the corrected tomographic image in the superimposed image shown in Figure 5(b), the normal tissue 50 and the tumor tissue 52, which are the form information, are corrected. Luminance of the tomographic image in the superimposed region is suppressed by the tomographic image correcting unit 40. Therefore, compared with the tumor tissue 52 superimposed in the tomographic image of the superimposed image shown in Figure 5(a), a texture of the tumor tissue 52 superimposed in the tomographic image of the superimposed image shown in Figure 5(b) is improved. When it is assumed that the tumor tissue 52 is displayed in black and the hardened tissue 54 is displayed in blue, a region where the tumor tissue 52 and the hardened tissue 54 are superimposed is displayed in deep blue. The operator can carefully observe the portion displayed in deep blue considering that the portion is highly likely to be a malignant tumor.

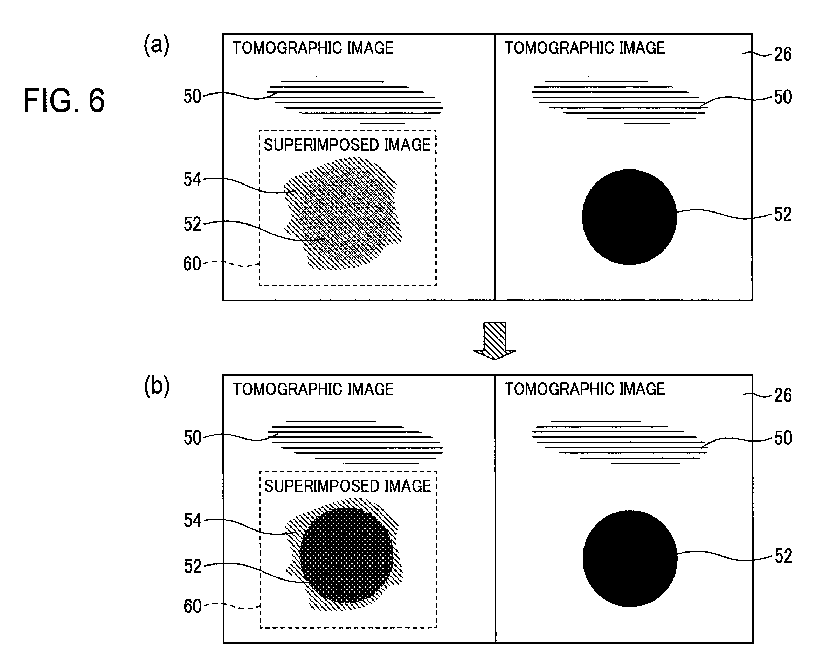

Figure 6 shows a display form of a superimposed image in which a display region of a tomographic image and a display region of an elasticity image are different. When the display region of the tomographic image and the display region of the elasticity image are different, the display region of the elasticity image is a superimposed region 60 of the superimposed image. When the display region of the tomographic image and the display region of the elasticity image are different, the superimposed region detecting unit 38 detects position information concerning the display region of the elasticity image as the superimposed region 60 of the superimposed image. The position information concerning the display region of the elasticity image set by the operation unit 42 is output to the superimposed region detecting unit 38. The superimposed region detecting unit 38 can also detect the position information of the display region of the elasticity image as the superimposed region 60 of the superimposed image. A calculated two-dimensional coordinate of the superimposed region 60 is communicated to the tomographic image correcting unit 40. The tomographic image correcting unit 40 corrects the tomographic image of the superimposed region 60 according to any one of the above methods shown in Figure 3, Figure 4, and the like.

Figure 6(a) shows a display form for displaying a superimposed image formed by an uncorrected tomographic image and an elasticity image and the tomographic image in parallel. Figure 6(b) shows a display form for displaying a superimposed image formed by a corrected tomographic image and an elasticity image and the tomographic image in parallel.

In the tomographic image shown in Figure 6(a), the normal tissue 50 and the tumor tissue 52, which are form information, are displayed. In the superimposed image shown in Figure 6(a), in addition to the normal tissue 50 and the tumor tissue 52, which are the form information, the hardened tissue 54, which is harness information of the superimposed region 60 of the superimposed image, is displayed to be superimposed on the tumor tissue 52 in the tomographic image.

In the tomographic image corrected in the superimposed region 60 of the superimposed image shown in Figure 6(b), the tumor tissue 52, which is the form information, is corrected. Luminance of the tomographic image in the superimposed region 60 of the superimposed image is suppressed by the tomographic image correcting unit 40. Therefore, compared with the tumor tissue 52 superimposed in the tomographic image of the superimposed image shown in Figure 6(a), a texture of the tumor tissue 52 superimposed in the tomographic image of the superimposed image shown in Figure 6(b) is improved without spoiling a texture of the normal tissue 50.

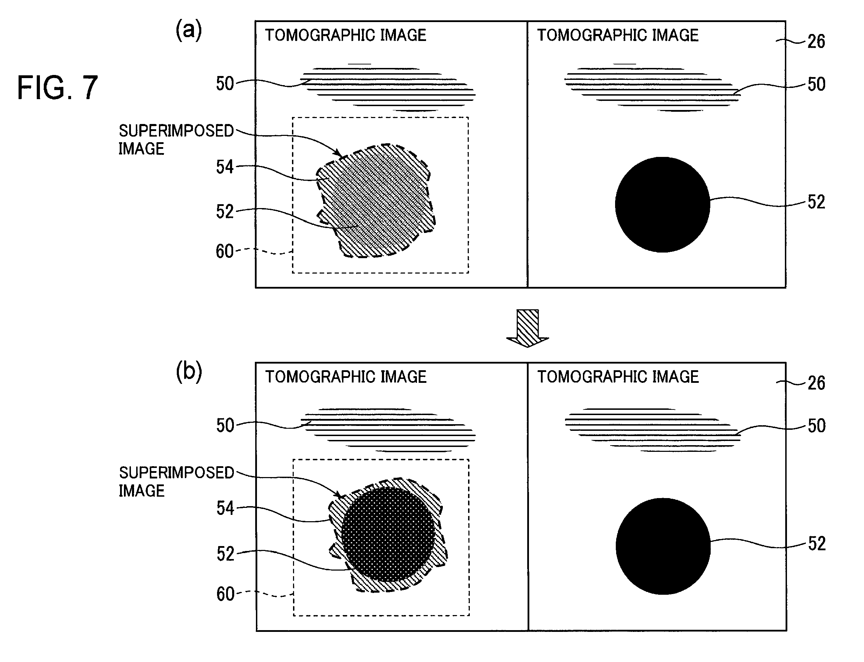

Figure 7 shows a display form in which a display region of an elasticity image is a superimposed region for a superimposed image. First, the elasticity information calculating unit 32 determines whether elasticity information (distortions or moduli of elasticity) concerning respective measurement points in a predetermined region 70 is a normally measured result. For example, the elasticity information calculating unit 32 compares the elasticity information concerning the respective measurement points and an average and a standard deviation of elasticity information in the predetermined region 70 and determines, on the basis of whether the elasticity information concerning the respective measurement points is normally measured, whether the elasticity information concerning the respective measurement points is valuable to be displayed. The elasticity image forming unit 34 forms an elasticity image based on the elasticity information while dividing the elasticity image into a region determined valuable to be displayed and a region determined not valuable to be displayed. For example, the elasticity image forming unit 34 forms the elasticity image only concerning the region determined valuable to be displayed.

The superimposed region detecting unit 38 detects, as a superimposed region for the superimposed image, position information concerning the display region of the elasticity image determined valuable to be displayed. The superimposed region detecting unit 38 detects, as the superimposed region 60 of the superimposed image, position information concerning the display region of the elasticity image determined valuable to be displayed.

A calculated two-dimensional coordinate of the superimposed region 60 is communicated to the tomographic image correcting unit 40. The tomographic image correcting unit 40 corrects the tomographic image of the superimposed region 60 according to any one of the above methods shown in Figure 3, Figure 4, and the like.

Figure 7(a) shows a display form for displaying a superimposed image formed by an uncorrected tomographic image and an elasticity image and the tomographic image in parallel. Figure 7(b) shows a display form for displaying a superimposed image formed by a corrected tomographic image and an elasticity image and the tomographic image in parallel.

In the tomographic image shown in Figure 7(a), the normal tissue 50 and the tumor tissue 52, which are form information, are displayed. In the superimposed image shown in Figure 7(a), in addition to the tumor tissue 52, which is the form information, the hardened tissue 54, which is hardness information of the superimposed region 60 of the superimposed image, is displayed to be superimposed on the tumor tissue 52 in the tomographic image.

In the tomographic image corrected in the superimposed region 60 of the superimposed image shown in Figure 7(b), the tumor tissue 52, which is the form information, is corrected. Luminance of the tomographic image in the superimposed region 60 of the superimposed image is suppressed by the tomographic image correcting unit 40. Therefore, compared with the tumor tissue 52 superimposed in the tomographic image of the superimposed image shown in Figure 7(a), a texture of the tumor tissue 52 superimposed in the tomographic image of the superimposed image shown in Figure 7(b) is improved without spoiling a texture of the normal tissue 50.

In this way, the tomographic image correcting unit 40 suppresses the luminance of the tomographic image in the superimposed region 60 of the superimposed image and does not suppress the luminance of the tomographic image outside the superimposed region 60 of the superimposed image. Therefore, the tomographic image in the superimposed region 60 is displayed at low luminance compared with the tomographic image outside the superimposed region 60.

As explained above, according to the first embodiment, there is provided the ultrasonic diagnostic device including the ultrasonic probe 12, the tomographic image forming unit 20 configured to form a tomographic image according to a reflected echo signal received via the ultrasonic probe 12, the elasticity image forming unit 34 configured to form an elasticity image on the basis of elasticity information calculated according to the reflected echo signal, and the image display unit 26 configured to display a superimposed image formed by the tomographic image and the elasticity image, the ultrasonic diagnostic device including the tomographic image correcting unit 40 configured to correct luminance of the tomographic image in a superimposed region for the tomographic image and the elasticity image. The tomographic image correcting unit 40 enhances the tomographic image in the superimposed region and diminishes the tomographic image in the superimposed region. The tomographic image correcting unit 40 changes a luminance distribution of the tomographic image in the superimposed region on the basis of a correction parameter for contributing a change of the luminance distribution of the tomographic image in the superimposed region. The ultrasonic diagnostic device includes the superimposed region detecting unit 38 configured to detect a superimposed region for the tomographic image formed by the tomographic image forming unit 20 and the elasticity image formed by the elasticity image forming unit 34. The superimposed region detecting unit 38 detects the superimposed region, which is a region where the tomographic image and the elasticity image are superimposed, from position information concerning a display region of the tomographic image and position information concerning a display region of the elasticity image.

Therefore, it is possible to improve visibility of the form information of the tomographic image in the superimposed region for the tomographic image and the elasticity image.

(Second Embodiment)

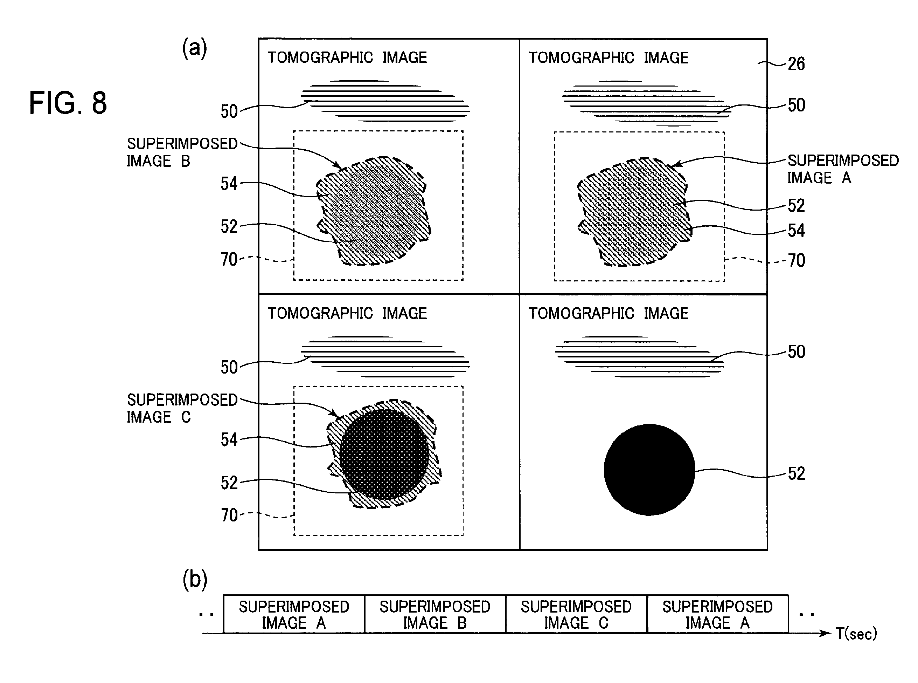

Next, a second embodiment is explained using Figure 8(a). The second embodiment is different from the first embodiment in that the image display unit 26 displays a plurality of superimposed images obtained by superimposing uniting a plurality of tomographic images corrected according to different correction parameters and an elasticity image.

For example, as explained with reference to Figures 3 and 4, a plurality of correction parameters (a correction luminance value, a correction coefficient, etc.) for a luminance distribution of a tomographic image in a superimposed region are set by the tomographic image correcting unit 40, whereby the image display unit 26 can display a plurality of superimposed images obtained by superimposing tomographic images having different luminance distributions and an elasticity image.

Here, a superimposed image obtained by superimposing uniting a tomographic image A corrected according to a correction luminance value A and an elasticity image is referred to as superimposed image A, a superimposed image obtained by superimposing uniting a tomographic image B corrected according to a correction luminance value B and the elasticity image is referred to as superimposed image B, and a superimposed image obtained by superimposing uniting a tomographic image C corrected according to a correction luminance value C and the elasticity image is referred to as superimposed image C. The image display unit 26 can display the superimposed image A, the superimposed image B, and the superimposed image C.

**EP 2 679 165 A1**

An operator selects any one of the superimposed images with the operation unit 42, whereby the image display unit 26 can display only the selected superimposed image or enlarge and display the selected superimposed image.

As explained above, according to the second embodiment, it is possible to check and improve visibility of form information of the tomographic image in the superimposed region for the tomographic image and the elasticity image.

(Third Embodiment)

Next, a third embodiment is explained using Figure 8(b). The third embodiment is different from the first and second embodiments in that the image display unit 26 displays, in order, superimposed images obtained by superimposing uniting a plurality of tomographic images corrected according to different correction parameters and an elasticity image.

For example, as explained with reference to Figures 3 and 4, a plurality of correction parameters (a correction luminance value, a correction coefficient, etc.) for a luminance distribution of a tomographic image in a superimposed region are set by the tomographic image correcting unit 40, whereby the image display unit 26 can display, in order, superimposed images obtained by superimposing uniting tomographic images having different luminance distributions and an elasticity image.

The image display unit 26 can repeatedly display the superimposed image A, the superimposed image B, and the superimposed image C in order. For example, as shown in Figure 8(b), the superimposed image B is displayed following the superimposed image A, the superimposed image C is displayed following the superimposed image B, and the superimposed image A is displayed following the superimposed image C. The image display unit 26 can display superimposed images in order from the superimposed image having a largest correction luminance value of a luminance distribution of a tomographic image in a superimposed region or display the superimposed images in order from the superimposed image having a smallest correction luminance value.

Concerning MPR display by a superimposed image of a tomographic image and an elasticity image, luminance of the tomographic image in a superimposed region can be corrected in the same manner. The MPR display is a display form for displaying orthogonal three cross sections. Concerning superimposed images of tomographic images and an elasticity image on the orthogonal three cross sections, it is possible to correct the tomographic images in superimposed regions according to respective correction parameters. That is, it is possible to respectively adjust, in a plurality of different cross sections, luminance of the tomographic images in the superimposed regions by the tomographic image correcting unit 40.

When a superimposed rendering image of a rendering image of a tomographic image and a rendering image of an elasticity image is displayed, it is possible to adjust luminance of the rendering image of the tomographic image in a superimposed volume region of the superimposed rendering image by the tomographic image correcting unit 40.

As explained above, according to the third embodiment, it is possible to check and improve visibility of form information of the tomographic image in the superimposed region for the tomographic image and the elasticity image.

Reference Signs List

1   Ultrasonic diagnostic device
10   Object
12   Ultrasonic probe
14   Transmitting unit
16   Receiving unit
17   Ultrasound transmission and reception control unit
18   Phasing addition unit
20   Tomographic image forming unit
24   Image superimposing unit
26   Image display unit
28   RF signal frame data selecting unit
30   Displacement measuring unit
32   Elasticity information calculating unit
34   Elasticity image forming unit
38   Superimposed region detecting unit
40   Tomographic image correcting unit
42   Operation unit
44   Image control unit

46    Pressure measuring unit

**Claims**

1. An ultrasonic diagnostic device comprising:

    an ultrasonic probe;
    a tomographic image forming unit configured to form a tomographic image according to a reflected echo signal received via the ultrasonic probe;
    an elasticity image forming unit configured to form an elasticity image on the basis of elasticity information calculated according to the reflected echo signal; and
    an image display unit configured to display a superimposed image formed by the tomographic image and the elasticity image,
    the ultrasonic diagnostic device comprising a tomographic image correcting unit configured to correct luminance of the tomographic image in a superimposed region for the tomographic image and the elasticity image.

2. The ultrasonic diagnostic device according to claim 1, wherein the tomographic image correcting unit enhances the tomographic image in the superimposed region and diminishes the tomographic image in the superimposed region.

3. The ultrasonic diagnostic device according to claim 1, wherein the tomographic image correcting unit changes a luminance distribution of the tomographic image in the superimposed region on the basis of a correction parameter for contributing a change of the luminance distribution of the tomographic image in the superimposed region.

4. The ultrasonic diagnostic device according to claim 3, wherein the tomographic image correcting unit sets the correction parameter for the tomographic image in the superimposed region on the basis of a superimposition ratio of the tomographic image and the elasticity image in the superimposed region.

5. The ultrasonic diagnostic device according to claim 1, wherein the tomographic image correcting unit corrects the tomographic image in the superimposed region on the basis of luminance information of the tomographic image.

6. The ultrasonic diagnostic device according to claim 1, further comprising:

    a tomographic image converting unit configured to convert the tomographic image output from the tomographic image correcting unit into monochrome information;
    a corrected tomographic image converting unit configured to convert the corrected tomographic image output from the tomographic image correcting unit into hue information; and
    an elasticity image converting unit configured to convert the elasticity image output from the elasticity image forming unit into hue information.

7. The ultrasonic diagnostic device according to claim 1, further comprising an adding unit configured to add up the corrected tomographic image and the elasticity image at a predetermined superimposition ratio and form the superimposed image.

8. The ultrasonic diagnostic device according to claim 1, further comprising a superimposed region detecting unit configured to detect the superimposed region for the tomographic image formed by the tomographic image forming unit and the elasticity image formed by the elasticity image forming unit.

9. The ultrasonic diagnostic device according to claim 8, wherein the superimposed region detecting unit detects, from position information concerning a display region of the tomographic image and position information concerning a display region of the elasticity image, the superimposed region that is a region where the tomographic image and the elasticity image are superimposed.

10. The ultrasonic diagnostic device according to claim 8, wherein, when the display region of the tomographic image and the display region of the elasticity image are the same, the superimposed region detecting unit detects the position information concerning the display region of the tomographic image as the superimposed region for the superimposed image.

**11.** The ultrasonic diagnostic device according to claim 8, wherein, when the display region of the tomographic image and the display region of the elasticity image are different, the superimposed region detecting unit detects the position information concerning the display region of the elasticity image as the superimposed region for the superimposed image.

**12.** The ultrasonic diagnostic device according to claim 8, wherein the superimposed region detecting unit detects, as the superimposed region for the superimposed image, position information of a display region of the elasticity image determined valuable to be displayed.

**13.** The ultrasonic diagnostic device according to claim 1, wherein the tomographic image correcting unit suppresses luminance of the tomographic image in the superimposed region and does not suppress luminance of the tomographic image outside the superimposed region.

**14.** The ultrasonic diagnostic device according to claim 1, wherein the tomographic image in the superimposed region is displayed at low luminance compared with the tomographic image outside the superimposed region.

**15.** An image display method comprising:

forming a tomographic image according to a reflected echo signal received via an ultrasonic probe;
forming an elasticity image on the basis of elasticity information calculated according to the reflected echo signal;
displaying a superimposed image formed by the tomographic image and the elasticity image; and
correcting luminance of the tomographic image in a superimposed region for the tomographic image and the elasticity image.

FIG. 1

# FIG. 2

**20** TOMOGRAPHIC IMAGE FORMING UNIT

**38** SUPERIMPOSED REGION DETECTING UNIT

**40** TOMOGRAPHIC IMAGE CORRECTING UNIT

**32** ELASTICITY INFORMATION CALCULATING UNIT

**34** ELASTICITY IMAGE FORMING UNIT

**44** IMAGE CONTROL UNIT

**42** OPERATION UNIT

**100** TOMOGRAPHIC IMAGE CONVERTING UNIT

**102** CORRECTED TOMOGRAPHIC IMAGE CONVERTING UNIT

**104** ELASTICITY IMAGE CONVERTING UNIT

**106**

**108** IMAGE SELECTING UNIT

**24**

**26** SUPERIMPOSED IMAGE / TOMOGRAPHIC IMAGE

# FIG. 3

## (a)

FREQUENCY

β

FREQUENCY

LUMINANCE
VALUE

A1    A2

LUMINANCE
VALUE

A3    A4

## (b)

FREQUENCY

FREQUENCY

LUMINANCE
VALUE

B1

LUMINANCE
VALUE

B1

EP 2 679 165 A1

FIG. 4

FIG. 5

(a)

| SUPERIMPOSED IMAGE | TOMOGRAPHIC IMAGE | ~26 |

50 — 〰〰〰 ... 50

54 — ...

52 — ... 52

(b)

| SUPERIMPOSED IMAGE | TOMOGRAPHIC IMAGE | ~26 |

50 — 〰〰〰 ... 50

54 — ...

52 — ... 52

EP 2 679 165 A1

FIG. 6

(a)

| TOMOGRAPHIC IMAGE | TOMOGRAPHIC IMAGE |

50 — 

SUPERIMPOSED IMAGE

54 —
52 —
60 —

26
50
52

(b)

| TOMOGRAPHIC IMAGE | TOMOGRAPHIC IMAGE |

50 —

SUPERIMPOSED IMAGE

54 —
52 —
60 —

26
50
52

EP 2 679 165 A1

FIG. 7

(a)

TOMOGRAPHIC IMAGE | TOMOGRAPHIC IMAGE — 26

50

SUPERIMPOSED
IMAGE

54

52

60

50

52

(b)

TOMOGRAPHIC IMAGE | TOMOGRAPHIC IMAGE — 26

50

SUPERIMPOSED
IMAGE

54

52

60

50

52

EP 2 679 165 A1

FIG. 8

## (a)

| TOMOGRAPHIC IMAGE | TOMOGRAPHIC IMAGE |

26

50

SUPERIMPOSED IMAGE B

54

52

70

SUPERIMPOSED IMAGE A

52

54

70

| TOMOGRAPHIC IMAGE | TOMOGRAPHIC IMAGE |

50

SUPERIMPOSED IMAGE C

54

52

70

50

52

## (b)

| . . | SUPERIMPOSED IMAGE A | SUPERIMPOSED IMAGE B | SUPERIMPOSED IMAGE C | SUPERIMPOSED IMAGE A | . . |

T(sec)

EP 2 679 165 A1

<table>
<tr><td colspan="2">**INTERNATIONAL SEARCH REPORT**</td><td colspan="2">International application No.<br>PCT/JP2012/000920</td></tr>
</table>

A.  CLASSIFICATION OF SUBJECT MATTER
*A61B8/08*(2006.01)i, *G06T1/00*(2006.01)i, *G06T3/00*(2006.01)i

According to International Patent Classification (IPC) or to both national classification and IPC

B.   FIELDS SEARCHED

Minimum documentation searched (classification system followed by classification symbols)
A61B8/08, G06T1/00, G06T3/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)

C.   DOCUMENTS CONSIDERED TO BE RELEVANT

| Category* | Citation of document, with indication, where appropriate, of the relevant passages | Relevant to claim No. |
|---|---|---|
| Y/A | JP 2004-135929 A  (Hitachi Medical Corp.),<br>13 May 2004 (13.05.2004),<br>& US 2006/0052702 A1    & EP 1554982 A1<br>& WO 2004/039262 A1    & CN 1705460 A<br>& CN 101427931 A | 1-3,5-15/4 |
| Y | JP 2011-19751 A  (GE Medical Systems Global<br>Technology Co., L.L.C.),<br>03 February 2011 (03.02.2011),<br>(Family: none) | 1-3,5-15 |
| Y | JP 2005-118152 A  (Hitachi Medical Corp.),<br>12 May 2005 (12.05.2005),<br>& US 2007/0032726 A1    & US 2009/0018444 A1<br>& US 2009/0149752 A1    & EP 1629777 A1<br>& WO 2004/105615 A1 | 8-12 |

☐  Further documents are listed in the continuation of Box C.       ☐  See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>01 March, 2012 (01.03.12) | Date of mailing of the international search report<br>13 March, 2012 (13.03.12) |
|---|---|
| Name and mailing address of the ISA/<br>Japanese Patent Office | Authorized officer |
| Facsimile No. | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004141505 A